(19) Europäisches Patentamt
European Patent Office
Office européen des brevets

(11) EP 0 874 801 B1

(12) **EUROPÄISCHE PATENTSCHRIFT**

(45) Veröffentlichungstag und Bekanntmachung des Hinweises auf die Patenterteilung:
**13.06.2001 Patentblatt 2001/24**

(21) Anmeldenummer: **96943040.4**

(22) Anmeldetag: **05.12.1996**

(51) Int Cl.7: **C07C 227/34**, C07B 57/00, C12P 41/00

(86) Internationale Anmeldenummer:
**PCT/EP96/05440**

(87) Internationale Veröffentlichungsnummer:
**WO 97/21663 (19.06.1997 Gazette 1997/26)**

(54) **VERFAHREN ZUR GEWINNUNG VON OPTISCH AKTIVEN L-ALPHA-AMINOCARBONSÄUREN AUS ENTSPRECHENDEN RACEMISCHEN D,L-ALPHA-AMINOCARBONSÄUREN**

PROCESS FOR OBTAINING OPTICALLY ACTIVE L-ALPHA-AMINOCARBOXYLIC ACIDS FROM CORRESPONDING RACEMIC D,L-ALPHA-AMINOCARBOXYLIC ACIDS

PROCEDE DE PRODUCTION D'ACIDES L-ALPHA-AMINOCARBOXYLIQUES OPTIQUEMENT ACTIFS PROVENANT DES ACIDES D,L-ALPHA-AMINOCARBOXYLIQUES RACEMIQUES CORRESPONDANTS

(84) Benannte Vertragsstaaten:
**AT BE CH DE DK ES FI FR GB IE IT LI NL PT SE**

(30) Priorität: **13.12.1995 DE 19546532**

(43) Veröffentlichungstag der Anmeldung:
**04.11.1998 Patentblatt 1998/45**

(73) Patentinhaber: **Degussa AG**
**40474 Düsseldorf (DE)**

(72) Erfinder:
- **DRAUZ, Karlheinz**
  **D-63579 Freigericht (DE)**
- **BOMMARIUS, Andreas**
  **D-60323 Frankfurt (DE)**
- **KARRENBAUER, Michael**
  **D-78345 Moos (DE)**
- **KNAUP, Günter**
  **D-63486 Bruchköbel (DE)**

(56) Entgegenhaltungen:
**EP-A- 0 175 840**
**EP-A- 0 232 486**
**DE-A- 2 344 060**
**DE-A- 2 419 838**

- **DATABASE WPI Week 9237 Derwent Publications Ltd., London, GB; AN 92-306693 XP002028872 "Prodn. of optically-active L-aminoacid(s)..." & SU 1 685 924 A (ARMN BRANCH CHEM REAGENTS ULTRAPURE) , 23.Oktober 1991**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 874 801 B1

Printed by Jouve, 75001 PARIS (FR)

## Beschreibung

Die Erfindung befaßt sich mit einem Verfahren zur Gewinnung von optisch aktiven L-α-Aminocarbonsäuren aus den entsprechenden racemischen D,L-α-Aminocarbonsäuren. Insbesondere beschäftigt sich die Erfindung mit einem Verfahren, bei dem

(a) die D,L-α-Aminocarbonsäuren acetyliert werden;

(b) die im Gemisch der dabei erhaltenen N-Acetyl-D,L-α-aminocarbonsäuren vorliegende N-Acetyl-L-α-aminocarbonsäure enzymatisch in die L-α-Aminocarbonsäure gespalten wird;

(c) die L-α-Aminocarbonsäure aus dem Gemisch abgetrennt wird unter Rückbehalt einer unter anderem N-Acetyl-D(L)-α-aminocarbonsäuren und eine der gebildeten L-α-Aminocarbonsäure äquivalenten Menge Acetat aufweisenden Lösung; und

(d) die in der Lösung enthaltene N-Acetyl-D(L)-α-aminocarbonsäure racemisiert und zur enzymatischen Spaltung zurückgeführt wird.

Die stereospezifische Spaltung von N-Acetyl-D,L-α-aminocarbonsäuren durch Aminoacylasen ist eines der effektivsten Verfahren zur Herstellung optisch aktiver α-Aminosäuren ausgehend von den entsprechenden racemischen D,L-α-Aminocarbonsäuren.

Diese Verfahren können sich im wesentlichen und insbesondere aus folgenden Teilschritten zusammensetzen:

1) Herstellung eines racemischen Gemisches der N-Acetyl-D,L-α-aminocarbonsäuren aus den racemischen D, L-α-Aminocarbonsäuren.

Das gebräuchlichste Verfahren zur Acetylierung von Aminocarbonsäuren nach dem Stand der Technik ist die Schotten-Baumann-Acetylierung. Hierzu wird eine wäßrige Lösung von D,L-α-Aminocarbonsäuren unter basischen Bedingungen mit Acetanhydrid oder Acetylchlorid versetzt. Die Isolierung der N-Acetyl-D,L-α-aminocarbonsäuren erfolgt nach Ansäuern durch Filtration oder Extraktion.

2) Enzymatische Spaltung der im racemischen Gemisch enthaltenen N-Acetyl-L-α-aminocarbonsäuren.

Für die enzymatische Spaltung werden die N-Acetyl-D,L-α-aminocarbonsäuren üblicherweise in Wasser gelöst und mit einer Base, vorzugsweise Natronlauge, auf einen pH-Wert zwischen etwa 6 und 8 gestellt, d. h. in die entsprechenden Salze der Carbonsäuren überführt. Mit Hilfe von Acylasen wird dann selektiv die N-Acetyl-L-α-aminocarbonsäure in Acetat und die L-α-Aminocarbonsäure gespalten. Aus dieser Reaktion resultiert eine Lösung, die neben Acetat und der Acylase unter anderem das angestrebte Zielprodukt, nämlich die L-α-Aminocarbonsäure, aber auch nicht umgesetzte N-Acetyl-D(L)-α-aminocarbonsäure, gegebenenfalls als Salz enthält. Die Schreibweise N-Acetyl-D(L)-α-aminocarbonsäure bezeichnet dabei eine Mischung, die überwiegend aus N-Acetyl-D-α-aminocarbonsäure besteht und die untergeordnete Mengen nicht gespaltener Reste N-Acetyl-L-α-aminocarbonsäure aufweist.

3) Trennung der L-α-Aminocarbonsäuren und der N-Acetyl-D(L)-α-aminocarbonsäuren.

Zur Gewinnung des Zielproduktes ist es erforderlich, dieses aus dem im vorhergehenden Schritt erhaltenen Gemisch abzutrennen. Im Falle der in Wasser relativ schwerlöslichen Aminosäuren, wie z. B. Phenylalanin, Tryptophan, Methionin, Valin, kann dies beispielsweise durch Filtration nach Aufkonzentration oder durch Filtration bei einer von Anfang an konzentrierten Fahrweise erfolgen.

4) Racemisierung und Rückführung der nicht umgesetzten N-Acetyl-D-α-aminocarbonsäuren.

Unter ökonomischen und ökologischen Aspekten ist es nicht nur erforderlich, die gebildete L-α-Aminocarbonsäure zu isolieren, sondern auch die N-Acetyl-D(L)-α-aminocarbonsäure und unter Umständen das Enzym abzutrennen und zu recyclieren. Die Abtrennung des Enzyms kann entweder durch Ultrafiltration der Reaktionslösung oder bei entsprechend konzentrierter Fahrweise durch Ultrafiltration einer Mutterlauge der Filtration, in der die L-α-Aminocarbonsäure isoliert wurde, erfolgen. Alternativ ist es ebenfalls möglich, bei Inkaufnahme von längeren Reaktionszeiten, die eingesetzte Enzymmenge soweit zu reduzieren, daß auf eine Abtrennung vollständig verzichtet werden kann.

Nicht verzichtet wird jedoch in der Regel auf die Racemisierung und Rückführung der nicht umgesetzten N-Acetyl-D(L)-α-aminocarbonsäuren. Für die Aufarbeitung des nach der Isolation der L-α-Aminocarbonsäure zurückbleibenden Gemisches hat es im Stand der Technik bereits eine Reihe von Vorschlägen gegeben.

Das Gemisch enthält neben weiteren Bestandteilen Salze der N-Acetyl-D(L)-α-aminocarbonsäuren, Acetat und Reste der L-α-Aminocarbonsäure. Eine Aufarbeitung ist u. a. entsprechend der JP OS 138603/76 durch Nachacetylierung der freien L-α-Aminocarbonsäure und nachfolgende Racemisierung des nachacetylierten Gemisches durch Zugabe von annähernd äquimolaren Mengen Acetanhydrid und Erhitzen des in Form einer Lösungs vorliegenden Gemisches möglich. Die Isolierung der relativ schwerlöslichen N-Acetyl-D,L-α-aminocarbonsäuren kann durch Ansäuern und geeignete Abtrennung, beispielsweise durch Filtration, erfolgen.

Vorteilhafter ist eine Aufarbeitung der Mutterlaugen aus der enzymatischen Spaltung wie in der DE 37 02 689 beschrieben. In diesem Fall kann auch die L-α-Aminocarbonsäure vollständig abgetrennt werden. Die Racemisierung der freien N-Acetyl-D(L)-α-aminocarbonsäuren kann dann, wie in der EP-A-0 175 840 beschrieben, in der Schmelze mit katalytischen Mengen Acetanhydrid erfolgen. Durch Lösen der racemisierten N-Acetyl-D,L-α-aminocarbonsäuren in Natronlauge resultiert ein Gemisch, das wieder zur Acylasespaltung eingesetzt werden kann.

Beim Verfahren gemäß der JP OS 138603/76 ist insbesondere nachteilig, daß für die dort beschriebene Racemisierung der N-Acetyl-D(L)-α-Aminocarbonsäure annähernd äquimolare Mengen Acetanhydrid benötigt werden.

Weiterhin stellt es einen erheblichen Nachteil dar, daß die N-Acetyl-D,L-α-aminocarbonsäure durch Kristallisation zurückgewonnen wird.

Die aus der DE 37 02 689 oder EP-A-0 175 840 bekannten Aufarbeitungsmethoden sind ebenfalls auf mehrfache Weise mit Nachteilen behaftet. So fällt bei der Aufarbeitung der Mutterlauge mittels Ionenaustauscher die N-Acetyl-D(L)-α-aminocarbonsäure als wäßrige Lösung an, die eine dem Natriumacetat entsprechende Menge Essigsäure enthält. Bedingt durch den sauren pH-Wert dieser Lösung tritt bei der anschließenden Eindampfung stets im geringen Umfang Hydrolyse zur freien α-Aminocarbonsäure ein, was bei der anschließenden Racemisierung zu einer erhöhten Bildung von Acyldipeptiden führt. Auch die in der EP-A 0 175 840 beschriebene Racemisierung der freien N-Acetyl-D(L)-α-aminocarbonsäure in der Schmelze mit katalytischen Mengen Acetanhydrid führt stets zur Bildung von Acetyldipeptiden.

Bei allen beschriebenen Verfahren ist zur Recyclierung der N-Acetyl-D(L)-α-aminocarbonsäuren die Überführung der in der Mutterlauge vorliegenden Salze in die freien Säuren erforderlich, was unweigerlich mit einem Salzanfall verbunden ist.

Angesichts der vorbeschriebenen Problematik und des hierin genannten und diskutierten Standes der Technik liegt der Erfindung die Aufgabe zugrunde, ein Verfahren zur Gewinnung optisch aktiver L-α-Aminocarbonsäuren aus entsprechenden racemischen D,L-α-Aminocarbonsäuren so fortzubilden, daß eine Recyclierung aller Einsatzstoffe möglich wird, wobei vor allem auch eine drastische Reduktion der Salzfracht angestrebt wird. Insbesondere soll das erfindungsgemäße Verfahren der Idealgleichung nahekommen, nach welcher Acetanhydrid und D,L-α-Aminocarbonsäuren vollständig zu L-α-Aminocarbonsäuren und Essigsäure umgesetzt werden.

Gelöst werden die genannten Aufgaben von einem Verfahren der eingangs erwähnten Gattung mit den Merkmalen des kennzeichnenden Teils des Anspruchs 1. Zweckmäßige Weiterbildungen der Erfindung werden in den von Anspruch 1 abhängigen Unteransprüchen unter Schutz gestellt.

Dadurch, daß die rückbehaltene Lösung aus Schritt c) so eingestellt wird, daß eine formal im wesentlichen aus N-Acetyl-D(L)-α-aminocarbonsäuresalz und freier Essigsäure im Gleichgewicht mit Acetat und freier N-Acetyl-D (L)-α-aminocarbonsäure bestehende Lösung erhalten wird, aus welcher Essigsäure destillativ abgetrennt wird, gelingt es in nicht ohne weiteres vorhersehbarer Weise, das mit der Erfindung angestrebte Ziel, nämlich eine der Idealvorstellung möglichst nahekommende Stoffbilanz, zu verwirklichen.

Eine wie angedeutet nur aus N-Acetyl-D(L)-α-aminocarbonsäuresalz und freier Essigsäure im Gleichgewicht mit Acetat und freier N-Acetyl-D(L)-α-aminocarbonsäure bestehende Lösung kann dem Grunde nach auf jede dem Fachmann geläufige Weise in einem Verfahren zur Gewinnung von optisch aktiven L-α-Aminocarbonsäuren erhalten werden. Im einfachsten Fall stellt die aus Schritt c) rückbehaltene Lösung eine solche "Mutterlauge" dar.

Falls die Abtrennung der L-α-Aminocarbonsäure aus dem Gemisch von Schritt c) nicht vollständig durchführbar ist, muß die in Lösung verbleibende L-α-Aminocarbonsäure zunächst nachacetyliert werden. Um die Menge an Acetat, die in der Lösung ("Mutterlauge") vorliegt, nicht weiter zu erhöhen, wird die Nachacetylierung zweckmäßig ohne Zusatz einer Base durchgeführt. D. h., mit der Nachacetylierung wird bevorzugt eine wässrige Lösung erhalten, die im wesentlichen nur noch N-Acetyl-D(L)-α-aminocarbonsäuresalz, Acetat und Essigsäure enthält.

Beim Kation des Salzes handelt es sich um das Gegenion der für eine pH-Einstellung in Schritt b) benötigten Base. Prinzipiell möglich sind u. a. Alkali, Erdalkali und Ammonium. Besonders zweckmäßig ist aus Kostengründen Natrium das Gegenion im N-Acetyl-D(L)-α-aminocarbonsäuresalz bzw. im Acetat.

Da die nachacetylierte Lösung aufgrund der Abtrennung der L-α-Aminocarbonsäure eine bezogen auf den Natriumacetatgehalt zu geringe Konzentration an N-Acetyl-D,L-α-aminocarbonsäure aufweist, ist es zweckmäßig, daß der Lösung nach der Nachacetylierung und vor der Eindampfung eine der abgetrennten L-α-Aminocarbonsäure äquimolare Menge N-Acetyl-D,L-α-aminocarbonsäure zugesetzt wird. Nach Zugabe einer der isolierten L-α-Aminocarbonsäure entsprechenden Menge N-Acetyl-D,L-α-aminocarbonsäure ergibt sich somit eine Lösung, die formal aus dem Salz der N-Acetyl-D(L)-α-aminocarbonsäure und Essigsäure besteht, die entsprechend der Acidität der Säuren im Gleichgewicht mit der freien N-Acetyl-D(L)-α-aminocarbonsäure und dem zugehörigen Salz der Essigsäure besteht.

Im Rahmen der Erfindung ist daher eine besonders günstige Verfahrensweise dadurch gekennzeichnet, daß der Lösung vor der destillativen Essigsäureabtrennung eine der abgetrennten L-α-Aminocarbonsäure äquimolare Menge N-Acetyl-D,L-α-aminocarbonsäure zugesetzt wird und daß die Lösung bei einem pH-Wert von zwischen 3 und 8, bevorzugt zwischen 4,5 und 5,5, bis zur Schmelze eingeengt wird. Hierzu wird am besten die Lösung auf geeignete Weise im Vakuum bei Temperaturen von etwa 100 bis 220 °C bis zur Schmelze eingedampft. Bevorzugt sind Temperaturen von 130 bis 180 °C. In den Fällen, in denen die Salze (bevorzugt die Natriumsalze) der N-Acetyl-D(L)-α-aminocarbonsäuren einen relativ hohen Schmelzpunkt besitzen, d. h. über etwa 200 °C, sind Temperaturen unterhalb der Schmelztemperatur ausreichend, da selbst in einer Mischung der N-Acetyl-D(L)-α-aminocarbonsäure- und Essigsäuresalze als Feststoffe im Vakuum Essigsäure unter Bildung der Salze der N-Acetyl-D(L)-α-aminocarbonsäuren abdestilliert werden kann.

Die bereits weiter oben angesprochene Nachacetylierung wird zweckmäßig so ausgeführt, daß die N-Acetyl-D,L-α-aminocarbonsäuren zur enzymatischen Spaltung in wäßrigem Medium in Salze überführt werden, und daß der Lösung vor Durchführung des Schrittes (d) zur Nachacetylierung in ihr verbliebener Reste an L-α-Aminocarbonsäuren ohne Zugabe weiterer Base mindestens 1,1 Mol-Äquivalente, bezogen auf die verbliebene Menge L-α-Aminocarbonsäuren, Acetanhydrid zugegeben wird. Hierdurch gelingt es auf verblüffend einfache Weise, die nach Abtrennung der L-α-Aminocarbonsäuren als "Mutterlauge" anfallende Lösung einer Verwendung im Kreis zuzuführen.

Im Rahmen der Erfindung wurde erstaunlicherweise gefunden, daß die Lösung, die als "Mutterlauge" aus einer Verfahrensvariante resultiert, bei welcher die durch die Acylase freigesetzte L-α-Aminocarbonsäure durch Filtration nach Aufkonzentrierung oder durch Filtration nach konzentrierter Fahrweise isoliert wird, und die neben dem N-Acetyl-D(L)-α-aminocarbonsäuresalz eine der gebildeten Menge L-α-Aminocarbonsäure äquimolare Menge Acetat sowie Reste der L-α-Aminocarbonsäure enthält, ohne Abtrennung der restlichen L-α-Aminocarbonsäure und ohne Abtrennung des Acetats in eine Form gebracht werden kann, die wieder direkt in der Acylasespaltung einsetzbar ist. Dadurch wird die Gewinnung optisch aktiver L-α-Aminocarbonsäuren aus den entsprechenden racemischen D,L-α-Aminocarbonsäuren deutlich effizienter gestaltet.

Die als "Mutterlauge" resultierende und nach erfindungsgemäßer Behandlung wieder zur Acylasespaltung einzusetzende Lösung kann dabei von wechselnder Zusammensetzung sein. So kann es sich bei der "Mutterlauge" um eine beim erstmaligen Anfahren des Prozesses anfallende Lösung handeln. Es ist aber ebenso möglich, daß als "Mutterlauge" eine nach mehrmaliger Kreisführung resultierende Lösung anfällt. Schließlich kann die "Mutterlauge" auch solche Lösungen umfassen, die durch Vereinigung verschiedener Lösungen, beispielsweise verschiedener aus parallelen enzymatischen Spaltschritten stammender Spaltlaugen enstehen.

In jedem Fall weist sie jedoch bevorzugt eine bestimmte Zusammensetzung auf. Und zwar liegt sie, nach einer bestimmten Behandlung in einer bestimmten Form vor. Die bestimmte Behandlung umfaßt das Einstellen der Lösung mit einer Base auf einen pH-Wert zwischen 4 und 8, so daß die N-Acetyl-D,L-α-aminocarbonsäuren in Form ihrer Salze in wäßriger Lösung vorliegen. Zweckmäßig ist ein pH-Wert zwischen 6 und 8. Günstig ist die Verwendung von verdünnter Natronlauge zur Einstellung des pH-Werts. Die Behandlung umfaßt ferner die selektive enzymatische Spaltung der N-Acetyl-L-α-aminocarbonsäuren und Abtrennung der L-α -Aminocarbonsäuren. Unmittelbar nach der Behandlung, d. h. vom Zeitpunkt her gesehen nach Abtrennung der L-α -Aminocarbonsäuren, ist die "Mutterlauge" mithin eine Lösung mit bevorzugt bis zu 5 Gew.-% nicht abgetrennter L-α -Aminocarbonsäure, bis zu 40 Gew.-% N-Acetyl-D(L)-α-aminocarbonsäuresalz und bis zu 15 Gew.-% Acetat, wobei sich alle Gewichtsangaben auf das Gesamtgewicht der wässrigen Lösung ("Mutterlauge") beziehen.

Ein weiterer Aspekt des erfindungsgemäßen Verfahren kann in der Überführung der dem Prozeß zuzuführenden N-Acetyl-D,L-α-aminocarbonsäure in das Natriumsalz unter Verwendung des in der "Mutterlauge" vorliegenden Natriumacetats bestehen.

Dies ist möglich, da im Rahmen der Erfindung unerwartet gefunden wurde, daß es gelingt, aus einer Lösung, die äquimolare Mengen N-Acetyl-D,L-α-aminocarbonsäure und Natriumacetat enthält, die Essigsäure fast vollständig abzudestillieren, ohne daß nachteilige Nebenreaktionen auftreten.

Um dieses Verfahren auf die aus der Racematspaltung resultierende "Mutterlauge" anzuwenden, ist es zunächst erforderlich, die in der "Mutterlauge" vorliegende freie Aminosäure zu acetylieren.

Besonders zweckmäßig und als besonders vorteilhaft hat sich die Durchführung der Nachacetylierung erwiesen, wenn diese durch Zugabe von mindestens 1,5 Äquivalenten, am besten zwischen 1,7 und 2,0 Äquivalenten, Acetanhydrid bezogen auf die in Lösung vorliegenden Restmengen an L-α-Aminocarbonsäure ohne Zusatz einer Base erfolgt.

Wird die Nachacetylierung bei weniger als 1,1 Äquivalenten Acetanhydridzugabe durchgeführt, besteht die Gefahr einer unvollständigen Überführung der L-α-Aminocarbonsäure(salze) in die N-acetylierte Verbindung. Wenn

mehr als 3,0 Äquivalente eingesetzt werden, ist die Stoffbilanz ungünstig, da zuviel Acetanhydrid ungenutzt bleibt. Darüber hinaus ist auch die Gefahr von unerwünschten Nebenreaktionen deutlich größer.

Da die Erfindung die Kreislaufführung aller Stoffe anstrebt, kann das nach Abtrennung der Essigsäure resultierende Stoffgemisch weiterverwertet werden. Besonders vorteilig kann das resultierende Salz der N-Acetyl-D(L)-aminosäure nach Einengung racemisiert und ohne weiteren Zusatz in Wasser gelöst und zur enzymatischen Racematspaltung rückgeführt werden. Gegenüber den bisher bekannten Verfahrensweisen hat die erfindungsgemäße Variante den großen Vorteil, daß nicht zunächst Natriumionen abgetrennt werden müssen, was in der Regel als Salz einer Mineralsäure geschieht, wobei die entfernten Natriummineralsäuresalze über das Abwasser zu entsorgen sind. Außerdem erspart die erfindungsgemäße Vorgehensweise die erneute Bildung von Salz, was bei literaturbekannten Verfahren dadurch provoziert wird, daß im Zuge der Neutralisation der N-Acetyl-D(L)-α-aminocarbonsäuren erneut Natriumionen in das System eingebracht werden. Gegenüber der Aufarbeitung über Ionenaustauscher hat das Verfahren der Erfindung den weiteren Vorteil, daß bei Eindampfung der Natriumsalze, d. h. bei einem pH-Wert zwischen 3 und 8, verglichen mit der Eindampfung einer N-Acetyl-D(L)-α-aminocarbonsäure/Essigsäure-Mischung, die einen pH-Wert von etwa zwei aufweist, die Hydrolyse der Acetylaminosäuren deutlich reduziert und die Nebenproduktbildung minimiert wird.

Die Racemisierung der aus dem erfindungsgemäßen Verfahren resultierenden N-Acyl-D(L)-aminosäuresalze ist grundsätzlich auf alle dem Fachmann geläufigen Arten möglich.

Bevorzugt wird sie in der Schmelze durch Erhitzen auf höhere Temperaturen als Raumtemperatur, vorzugsweise unter Zusatz von katalytischen Mengen Acetanhydrid, durchgeführt. Im Vergleich zu der in der EP 0 175 840 beschriebenen Racemisierung der freien N-Acetyl-D(L)-aminosäuren werden dabei deutlich weniger Nebenprodukte (insbes. acetylierte Dipeptide) gebildet.

Weiterhin ist es möglich, die Racemisierung der Acetyl-D(L)-aminosäure-Salze wie in der JP OS 138603/76 beschrieben, in Lösung durchzuführen.

In diesem Falle wird die Racemisierung am vorteilhaftesten vor der Zugabe der N-Acetyl-D,L-α-aminosäure und vor dem Einengen zur Schmelze durchgeführt.

Das erfindungsgemäße Verfahren kann auch vorteilhaft bei der Aufarbeitung mittels Ionenaustauscher anfallenden wäßrigen Lösung aus N-Acetyl-D(L)-α-aminosäure und Essigsäure angewandt werden.

Nachdem die Neutralisation gemäß dem Stand der Technik zeitlich nach der Racemisierung (nach Ablauf der Verweilzeit) durch Abschrecken mit einer wässrigen Alkalimetallhydroxid- oder Ammoniaklösung erreicht wurde, ist die mit der Überführung zumindest eines Teils der N-Acetyl-D(L)-α-aminocarbonsäuren in die entsprechenden Salze neben einer Reduzierung der Hydrolyse bei der Eindampfung auch durch die damit verbundene Änderung der Zusammensetzung des zu racemisierenden Gemisches in nicht vorhersehbarer Weise mit verantwortlich für eine günstige Gestaltung der Nebenproduktbildung.

Insbesondere kann vermutet werden, daß dies auf eine deutlich erhöhte thermische Beständigkeit der Salze im Vergleich zu den freien Acetylaminosäuren zurückzuführen sein könnte.

Im Rahmen der Erfindung wird die Racemisierung der N-Acetyl-D(L)-α-aminocarbonsäuresalze oder eines Gemisches, das diese Salze zusammen mit den entsprechenden freien Säuren aufweist, besonders zweckmäßig im "nicht-wässrigen" Zustand durchgeführt. Hierunter wird erfindungsgemäß verstanden, daß die Racemisierung nicht in wässriger Lösung durchzuführen ist. Die absolute Wasserfreiheit ist jedoch, wie bereits oben erwähnt, nicht Voraussetzung für die erfolgreiche Durchführung des Verfahrens gemäß der Erfindung.

Unter Überführung wenigstens eines Teils der N-Acetyl-D(L)-α-aminocarbonsäuren in die dazugehörigen N-Acetyl-D(L)-α-aminocarbonsäuresalze wird verstanden, daß bei der Racemisierung selbst ein Gemisch aus freier Säure und Salz oder aber nur das Salz vorliegt.

Sofern ein Gemisch aus Salz und Säure für die Racemisierung verwendet wird, kann die Racemisierungsmischung beispielsweise durch Mischen der entsprechenden reinen Substanzen erhalten werden.

In bevorzugter erfindungsgemäßer Ausführungsform wird jedoch eine wäßrige Lösung der N-Acetyl-D(L)-α-aminocarbonsäure ggf. auch in Gegenwart von Essigsäure mit einer Base (vorzugsweise NaOH) versetzt. Hierbei wird ein pH-Wert erreicht oder eingestellt, der nicht über 8 liegt.

Vorzugsweise wird eine wäßrige Lösung der N-Acetyl-D(L)-α-aminocarbonsäure oder eine diese im Gemisch mit einem entsprechenden N-Acetyl-D(L)-α-aminocarbonsäuresalz enthaltende wäßrige Lösung auf einen pH-Wert von 2 - 8, bevorzugt 4 - 8, besonders bevorzugt 4,5 - 5,5, gestellt.

Durch die Einstellung auf einen bestimmten pH-Bereich oder -Wert wird vorteilhaft nicht nur die wesentliche Menge der freien Säure in das zugehörige Salz überführt, gleichzeitig wird durch das dabei eingestellte Verhältnis auch ein für die thermische Racemisierung günstiges Verhältnis der interessierenden Reaktionsgeschwindigkeiten erreicht. Der bei dem erfindungsgemäßen Verfahren eingestellte pH-Wert von 2 - 8, vorteilhaft von 4 - 8 und vorzugsweise 4,5 - 5,5, reduziert die Hydrolysegefahr der N-Acetyl-D(L)-α-aminocarbonsäuresalze im Vergleich zu der freien N-Acetylaminosäure erheblich und damit die Nebenproduktbildung (N-Acetyldipeptide), so daß selbst längere Verweilzeiten bzw. höhere Temperaturen nahezu keinen negativen Einfluß auf die Ausbeute haben. Der pH-Wert wird vorzugsweise auf 4,5 - 5,5 eingestellt, weil in diesem pH-Bereich die Pufferwirkung der Mischung am größten ist.

Zur Einstellung des gewünschten pH-Wertes kann grundsätzlich jede dem Fachmann geläufige Base Verwendung finden, die geeignete Salze mit N-Acetyl-D(L)-α-aminocarbonsäuren bilden kann. Bevorzugt werden die Alkali-Laugen. Ganz besonders bevorzugt ist NaOH, weil die pH-Einstellung mit Natronlauge im technischen Maßstab wesentlich erleichtert wird.

Die mit einer Base auf den erfindungsgemäßen pH-Wert-Bereich eingestellte Lösung wird in einer nützlichen Verfahrensmodifikation unter Erhalt eines Rückstandes bis zur Trockne, also zum Erhalt eines Feststoffs, oder zur Schmelze eingedampft.

Dabei ist es von Vorteil, wenn dem erhaltenen Rückstand, sei es als Schmelze oder als trockener Feststoff, zur Racemisierung durch Erhitzen Acetanhydrid zugesetzt wird.

Handelt es sich bei der zu racemisierenden Substanz um eine Schmelze, so ist der Zusatz einer katalytischen Menge an Acetanhydrid bevorzugt, weil dadurch die Geschwindigkeit der Racemisierung deutlich gesteigert werden kann.

Das Aufschmelzen der N-Acetyl-D(L)-α-aminocarbonsäure und das Erhitzen der Schmelze erfolgt vorzugsweise unter Stickstoff. Das Aufschmelzen und das Erhitzen der Schmelze können jedoch auch ohne Schutzgas oder im Vakuum vorgenommen werden.

Obwohl die Verwendung einer Schmelze zur Racemisierung sehr vorteilhaft ist, hat es sich weiterhin unerwartet gezeigt, daß es für die Durchführung des erfindungsgemäßen Verfahrens zur Racemisierung nicht unbedingt erforderlich ist, eine Schmelze der Acetylaminosäuresalze zu erhalten. Im Falle, daß der Schmelzpunkt des Salzes sehr hoch liegt, ist es möglich, den Feststoff mit der erforderlichen Menge Acetanhydrid zu vermischen und diese Mischung auf Temperaturen unter dem Schmelzpunkt der Acetylaminosäuresalze zu erhitzen. Durch das zugesetzte Acetanhydrid kann sich eine partielle Schmelze bilden, die zur Racemisierung ausreichend ist.

Nach diesem Verfahren können z. B. Acetyl-phenylalaninnatrium und Acetyl-valin-natrium bereits bei Temperaturen von 160 °C racemisiert werden.

Die Menge an zuzusetzendem Acetanhydrid ist im Rahmen der Erfindung nicht besonders kritisch. Bevorzugt versetzt man die Schmelze oder partielle Schmelze unter guter Durchmischung mit 2 - 10 Gew.-%, besonders bevorzugt 2 - 6 Gew.-%, Acetanhydrid, bezogen auf die Summe aus N-Acetyl-D(L)-α-aminocarbonsäure und N-Acetyl-D(L)-α-aminocarbonsäuresalz.

Die Temperatur, die zur Racemisierung eingesetzt wird, soll möglichst hoch sein, ohne die Substanzen übermäßig zu schädigen oder Nebenreaktionen in den Vordergrund treten zu lassen.

Ist unter diesen Voraussetzungen der Erhalt einer Schmelze möglich, so wird die Schmelze zur Racemisierung vorteilhaft auf und bei einer Temperatur erhitzt, die 5 bis 10 °C über der Schmelztemperatur der jeweiligen N-Acetyl-D(L)-α-aminocarbonsäure oder des dazugehörigen Salzes liegt, je nachdem, welche Temperatur höher ist.

Ist aufgrund des Schmelzpunktes der beteiligten Substanzen der Erhalt einer vollständigen Schmelze nicht möglich, so ist es günstig, die Temperaturen zur Racemisierung im Bereich von 100 bis 220 °C, vorzugsweise 130 - 180 °C, zu wählen. Diese Bereiche erfüllen in der Regel alle zu stellenden Anforderungen.

Zur Weiterverarbeitung des Racemats kann man jede dem Fachmann hierfür geläufige Verfahrensvariante verwenden. Bevorzugt werden die Schmelze oder partielle Schmelze nach Beendigung des Erhitzens in Wasser aufgenommen und dann der weiteren Verarbeitung zugeführt.

Nach dem erfindungsgemäßen Verfahren lassen sich eine große Zahl α-Aminocarbonsäuren gewinnen. Zu den erfolgreich gewinnbaren Verbindungen gehören u. a. Verbindungen der Formel I

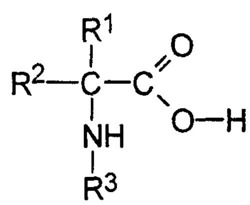

I,

worin

$R^1$ Wasserstoff oder $C_{1-4}$-Alkyl,

$R^2$ Wasserstoff, Alkyl, substituiertes Alkyl, Aryl, substituiertes Aryl, Heteroaryl, Arylalkyl, Heteroarylalkyl, Cycloalkyl- oder Cycloalkylalkyl ist, wobei die genannten Reste jeweils wiederum selbst substituiert sein können und/oder Heteroatome enthalten können,
oder

$R^1$ und $R^2$ zusammen mit dem sie verbindenden C-Atom einen 3 bis 7-gliedrigen gesättigten Ring bilden,
$R^3$ Wasserstoff oder $C_{1-4}$-Alkyl ist
oder
$R^2$ und $R^3$ zusammen mit dem sie verbindenden N- und C-Atom einen 4 bis 7-gliedrigen gesättigten Ring bilden, der ggf. Heteroatome enthalten kann.

Alkylgruppen können geradkettig oder verzweigt sein, bevorzugt sind Kettenlängen von $C_1$-$C_{12}$ bei geraden Ketten bzw. Kettenlängen von $C_{3-12}$ bei verzweigten Ketten, besonders bevorzugt Kettenlängen von $C_{1-6}$ bei geraden Ketten bzw. $C_{3-6}$ bei verzweigten Ketten, Beispiele sind Methyl, Ethyl, Propyl, Isopropyl, Butyl, Isooctyl, Dodecyl.

Diese Alkylgruppen sind bevorzugt substituiert durch 1 - 3 Amino-, Hydroxyl-, Halogen-, Guanidino-, Harnstoff-, Carboxy-, Carboxamid- und/oder Alkoxycarbonyl-Gruppen.

Arylgruppen können bevorzugt Phenyl- oder substituierte Phenyl-Gruppen sein.

Substituierte Arylgruppen sind bevorzugt Mono-, Di-, oder Trihalogen, Mono-, Di-, oder Trihydroxy, Mono-, Di-, Trialkyl-Phenylgruppen, wobei Halogen Fluor Chlor oder Brom ist, Alkyl $C_{1-4}$-Alkyl, bevorzugt Methyl oder Ethyl.

Heteroaryl-Gruppen sind bevorzugt 5 oder 6-Ring-Systeme mit 1 - 2 Heteroatomen im Ring, die O, N, oder S sein können.

Arylalkyl ist bevorzugt Benzyl. Cycloalkyl und Cycloalkylmethyl sind bevorzugt $C_{3-7}$-Ring-Systeme.

Bevorzugt sind Alanin, Valin, Leucin, Isoleucin, Prolin, Tryptophan, Phenylalanin, Methionin, Serin, Tyrosin, Threonin, Cystein, Asparagin, Glutamin, Histidin, Cystin, Citrullin, Homocystein, Homoserin, Hydroxyprolin, Ornithin, Norvalin sowie Derivate der vorstehend genannten Aminosäuren. Ganz besonders bevorzugt sind Methionin, Valin, Phenylalanin und/oder Norvalin. Besonders hervorzuheben ist die Gewinnung von L-Methionin.

Die Erfindung wird durch die nachfolgenden Beispiele näher erläutert. Alle Prozentangaben bedeuten, wenn nicht anders angegeben, Gewichtsprozente.

Beispiel 1: Herstellung von N-Acetyl-D,L-methionin

Unterschiedliche Mengen D,L-Methionin wurden in 100 ml Essigsäure gelöst, die Lösung im Ölbad temperiert und 1.1 Äquivalente Acetanhydrid zugegeben. Nach 5 min wurden die Ansätze im Vakuum bei 100 °C Badtemperatur eingeengt. Der Gehalt an N-Acetyl-D,L-methionyl-D,L-methionin (Ac-Met-Met) wurde per HPLC bestimmt. Die Ergebnisse dieser Versuche sind in der folgenden Tabelle zusammengefaßt:

| D,L-Methionin | Essigsäure | Acetanhydrid | Temp. | Ac-Met-Met |
|---|---|---|---|---|
| 7.5 g (50 mmol) | 100 ml | 5.6 g (55 mmol) | 80 °C | 1.8 % |
| 7.5 g (50 mmol) | 100 ml | 5.6 g (55 mmol) | 95 °C | 1.8 % |
| 14.9 g (100 mmol) | 100 ml | 11.2 g (110 mmol) | 95 °C | 3.2 % |
| 26.1 g (175 mmol) | 100 ml | 19.6 g (192 mmol) | 95 °C | 5.9 % |
| 37.3 g (250 mmol) | 100 ml | 28.1 g (275 mmol) | 95 °C | 7.8 % |

Beispiel 2: Herstellung von N-Acetyl-D,L-methionin unter technischen Bedingungen

373 g (2.5 mol) D,L-Methionin wurden in 5 l Essigsäure bei 90 °C gelöst und mit 281 g (2.75 mol) Acetanhydrid versetzt. Die Temperatur stieg dabei auf 95 °C an. Nach 10 min Nachreaktion wurde die Lösung an einem Sambay-Verdampfer bei 170 °C und 9 mbar eingeengt. Man erhielt 427 g eines zähen Öls, das beim Erkalten erstarrte. Nach HPLC-Analyse bestand das Produkt aus 92.3 % Acetyl-D,L-Methionin und 3.4 % Ac-Met-Met.

Beispiel 3: Herstellung von N-Acetyl-D,L-valin

11.7 g (0.10 mol) D,L-Valin wurden in 100 ml Essigsäure gelöst und bei 90 °C mit 11.2 g (0.11 mol) Acetanhydrid versetzt. Nach 10 min wurde bei 15 mbar und 100 °C eingeengt. Nach HPLC-Analyse bestand der kristalline Rückstand aus 95.6 % Acetyl-D,L-valin und 2.8 % N-Acetyl-D,L-valyl-D,L-valin.

Beispiel 4: Herstellung von N-Acetyl-D,L-Norvalin

11.7 g (0.10 mol) D,L-Norvalin wurden analog Beispiel 3 umgesetzt. Der kristalline Rückstand bestand nach HPLC-Analyse aus 95.2 % N-Acetyl-D,L-norvalin und 4.1 % N-Acetyl-D,L-norvalyl-D,L-norvalin.

Beispiel 5: Entfernen von Essigsäure aus einer Mischung von aus N-Acetyl-D,L-methionin und Natriumacetat

Jeweils 38.2 g (0.20 mol) N-Acetyl-D,L-methionin und 16.4 g (0.20 mol) Natriumacetat wurden intensiv gemischt und erhitzt. Ab ca. 100 °C bildeten sich Schmelzen. Die Schmelze wurde bei 15 mbar 30 min auf 150 - 180 °C erhitzt und nach Abkühlen der Gehalt an Essigsäure bestimmt. Die Ergebnisse sind in der folgenden Tabelle zusammengefaßt:

| Temperatur | Essigsäure |
|---|---|
| 150 °C | 5.3 % |
| 160 °C | 4.3 % |
| 170 °C | 3.0 % |
| 180 °C | 3.0 % |

Beispiel 6: Entfernen von Essigsäure aus einer Mischung von aus N-Acetyl-L-valin und Natriumacetat

79.6 g (0.50 mol) N-Acetyl-L-valin und 41.0 g (0.5 mol) Natriumacetat wurden unter Erwärmen in 150 ml Wasser gelöst und bei 100 °C und 15 mbar zur Trockne eingeengt. Teile des festen Rückstandes wurden anschließend für jeweils 30 min bei verschiedenen Temperaturen getrocknet und der Gehalt an Essigsäure bestimmt. Die Ergebnisse sind in der folgenden Tabelle zusammengefaßt:

| Temperatur | Essigsäure |
|---|---|
| 100 °C | 10.4 % |
| 150 °C | 5.5 % |
| 175 °C | 2.1 % |
| 200 °C | 1.7 % |

Beispiel 7: Entfernen von Essigsäure aus einer Mischung von aus N-Acetyl-L-phenylalanin und Natriumacetat

103.6 g (0.50 mol) N-Acetyl-L-phenylalanin und 41.0 g (0.5 mol) Natriumacetat wurden unter Erwärmen in 150 ml Wasser gelöst und bei 100 °C und 15 mbar zur Trockne eingeengt. Teile des festen Rückstandes wurden anschließend für jeweils 30 min bei verschiedenen Temperaturen getrocknet und der Gehalt an Essigsäure bestimmt. Die Ergebnisse sind in der folgenden Tabelle zusammengefaßt:

| Temperatur | Essigsäure |
|---|---|
| 100 °C | 7.3 % |
| 150 °C | 3.5 % |
| 175 °C | < 0.5 % |

Beispiel 8: Entfernen von Essigsäure aus einer Mischung von aus N-Acetyl-D,L-Norvalin und Natriumacetat

Jeweils 3.12 g (0.02 mol) N-Acetyl-D,L-Norvalin und 1.64 g (0.02 mol) Natriumacetat wurden intensiv gemischt und erhitzt. Ab ca. 110 °C bildete sich eine Schmelze. Die Schmelze wurde bei 15 mbar 30 min auf 160 °C erhitzt. Nach dem Abkühlen enthielt die Probe 2.2 % Essigsäure.

Beispiel 9: Vergleich der thermischen Beständigkeit von N-Acetyl-D,L-methionin und N-Acetyl-D,L-methionin-natriumsalz

Jeweils 20 g N-Acetyl-D,L-methionin und N-Acetyl-D,L-methionin-natriumsalz wurden auf 150 °C erhitzt. Die Bildung von N-Acetyl-D,L-methionyl-D,L-methionin wurde mit Hilfe der HPLC verfolgt. Die Ergebnisse sind in der folgenden Tabelle zusammengefaßt:

| Zeit | Ac-Met-OH | Ac-Met-ONa |
|---|---|---|
| 1 h | 1.7 % | 0.1 % |
| 2 h | 3.8 % | 0.2 % |
| 5 h | 8.8 % | 0.4 % |

Beispiel 10: Racemisierung von N-Acetyl-D(L)-methioninnatrium in der Schmelze

Jeweils 10 g (0.053 mol) N-Acetyl-D(L)-methionin wurden mit 8 gew.-%iger Natronlauge auf unterschiedliche pH-Werte gestellt und im Vakuum bei 50 mbar bis zur Schmelze eingedampft. Anschließend wurden die Schmelzen erhitzt und unter guter Rührung Essigsäureanhydrid zudosiert. Diese Schmelze wurde dann für eine bestimmte Zeit bei dieser Temperatur belassen und anschließend in Wasser aufgenommen. Zur Verfolgung der Racemisierung wurde der Drehwert ($[\alpha]_D^{20}$, c = 1 in Wasser) bestimmt. Vor der Racemisierung betrug dieser Drehwert +21.3. Die Ergebnisse dieser Versuche sind in der folgenden Tabelle zusammengefaßt:

| pH-Wert | $Ac_2O$ | Temp. | Verweilzeit | $[\alpha]_D^{20}$ | Ac-Met-Met |
|---|---|---|---|---|---|
| 5 | 2 % | 155 °C | 30 min | 0 | 0.3 % |
| 4 | 2 % | 155 °C | 30 min | +0.2 | 0.8 % |
| 6 | 2 % | 155 °C | 30 min | 0 | 0.2 % |
| 5 | 3 % | 110 - 120 °C | 30 min | +0.5 | 0.05 % |
| 5 | 2 % | 180 °C | 10 min | 0 | 0.1 % |
| 5 | 2 % | 180 °C | 30 min | 0 | 0.8 % |
| 8 | 3 % | 155 °C | 30 min | 0 | 0.4 % |
| 5 | 6 % | 155 °C | 30 min | 0 | 0.5 % |
| 1.6 | 2 % | 155 °C | 30 min | 0 | 5.5 % |

Beispiel 11: Racemisierung von N-Acetyl-L-phenylalaninnatriumsalz

Entsprechend Beispiel 7 bereitete und bei 150 °C im Vakuum getrocknete Proben von N-Acetyl-L-phenylalaninnatriumsalzen wurden mit Actanhydrid vermischt und erwärmt. Zur Verfolgung der Racemisierung wurde der Drehwert ($[\alpha]_D^{20}$, c = 1 in 1 N HCl) bestimmt. Vor der Racemisierung betrug dieser Drehwert +21.6. Die Ergebnisse sind in der folgenden Tabelle zusammengefaßt:

| $Ac_2O$ | Temp. | Verweilzeit | $[\alpha]_D^{20}$ |
|---|---|---|---|
| 2 % | 140 °C | 15 min | 9.0 |
| 2 % | 160 °C | 15 min | 2.9 |
| 2 % | 180 °C | 15 min | 1.9 |
| 5 % | 160 °C | 15 min | 0 |
| - | 230 °C | 15 min | 10.8 |

Beispiel 12: Racemisierung von N-Acetyl-L-valinnatriumsalz

Entsprechend Beispiel 6 bereitete und bei 150 °C im Vakuum getrocknete Proben von N-Acetyl-L-valin-natriumsalzen wurden mit Acetanhydrid vermischt und erwärmt. Zur Verfolgung der Racemisierung wurde der Drehwert ($[\alpha]_D^{20}$, c = 1 in 1 N HCl) bestimmt. Vor der Racemisierung betrug dieser Drehwert +11.9. Die Ergebnisse sind in der folgenden Tabelle zusammengefaßt:

| $Ac_2O$ | Temp. | Verweilzeit | $[\alpha]_D^{20}$ |
|---|---|---|---|
| 2 % | 140 °C | 15 min | 10.2 |
| 2 % | 160 °C | 15 min | 6.5 |
| 2 % | 180 °C | 15 min | 4.8 |
| 5 % | 160 °C | 15 min | 1.6 |
| 5 % | 230 °C | 15 min | 0 |
| - | 230 °C | 15 min | 8.3 |

Beispiel 13: Nachacetylierung einer N-Acetyl-D(L)-methionin-Mutterlauge

Je 100 g einer nach Abtrennung von L-Methionin durch Filtration erhaltenen Mutterlauge, die pro 100 g 8.3 g (0.100 mol) Natriumacetat, 19.3 g (0.089 mol) N-Acetyl-D(L)-methionin-natrium und 1.7 g (0.011 mol) L-Methionin enthielt, wurde bei verschiedenen Temperaturen mit unterschiedlichen Mengen Acetanhydrid versetzt. Nach jeweils 30 min wurden Proben entnommen und der Methioningehalt mit Hilfe der HPLC bestimmt. Die Ergebnisse sind in der folgenden Tabelle zusammengefaßt:

| Temp. | Acetanhydrid | Methionin |
|---|---|---|
| 30 °C | - | 9.0 Fl.-% |
| 30 °C | 1.16 g (11.4 mmol) | 2.9 Fl.-% |
| 30 °C | 1.74 g (17.1 mmol) | 1.2 Fl.-% |
| 30 °C | 2.03 g (19.9 mmol) | 0.6 Fl.-% |
| 30 °C | 2.61 g (25.6 mmol) | 0.2 Fl.-% |
| 60 °C | 1.16 g (11.4 mmol) | 2.9 Fl.-% |
| 60 °C | 1.74 g (17.1 mmol) | 0.2 Fl.-% |
| 60 °C | 2.03 g (19.9 mmol) | 0.0 Fl.-% |
| 90 °C | 1.16 g (11.4 mmol) | 2.9 Fl.-% |
| 90 °C | 1.74 g (17.1 mmol) | 0.3 Fl.-% |
| 90 °C | 2.03 g (19.9 mmol) | 0.1 Fl.-% |

Beispiel 14: Recyclierung einer N-Acetyl-D(L)-Methionin-natriumsalz-Mutterlauge

717 g (3.75 mol) N-Acetyl-D,L-methionin und 140 g (3.50 mol) Natriumhydroxid wurden in Wasser gelöst und auf 1.5 l aufgefüllt. Der pH-Wert der Lösung wurde mit 50 %iger Natronlauge auf 7 gestellt. Nach Zugabe von 3.6 g Acylase (Aktivität 31.000 E/g) wurde 5 Tage bei Raumtemperatur gerührt. Die Suspension wurde auf 5 °C abgekühlt, der Niederschlag abfiltriert, mit 300 ml Eiswasser gewaschen und getrocknet. Man erhielt 174 g (1.17 mol) L-Methionin. Das Filtrat (1800 g), das neben 21.8 Gew.-% N-Acetyl-D(L)-methionin 2.8 Gew.-% L-Methionin enthielt, wurde auf 60 °C erwärmt und mit 60 g (0.60 mol) Acetanhydrid versetzt. Nach 30 min war in der Lösung kein Methionin nachweisbar. Es wurden 223 g (1.17 mol) N-Acetyl-D,L-methionin zugegeben und im Vakuum auf 1200 g eingeengt. Diese Lösung wurde mit einem Sambay-Verdampfer bei 170 °C und 10 mbar auf 752 g eingeengt. In die noch warme Schmelze wurden 15 g Acetanhydrid eingerührt, 10 min auf 140 °C erhitzt und dann in Wasser gelöst und auf 1.5 l aufgefüllt. Der Drehwert der Lösung war Null. Der Gehalt an N-Acetyldipeptid betrug 0.8 % bez. auf N-Acetyl-methionin. Nachdem der pH-Wert mit Natronlauge auf 7 gestellt wurde, wurde diese Lösung wie oben beschrieben erneut zur Acylasespaltung eingesetzt. Man erhielt 166 g (1.11 mol) L-Methionin.

Beispiel 15: Recyclierung einer N-Acetyl-D(L)-Norvalinnatriumsalz-Mutterlauge

597 g (3.75 mol) N-Acetyl-D,L-norvalin und 140 g (3.50 mol) Natriumhydroxid wurden in Wasser gelöst und auf 1.5 l aufgefüllt. Der pH-Wert der Lösung wurde mit 50 %iger Natronlauge auf 7 gestellt. Nach Zugabe von 3.0 g

Acylase (Aktivität 31.000 E/g) wurde 5 Tage bei Raumtemperatur gerührt. Die Suspension wurde auf 5 °C abgekühlt, der Niederschlag abfiltriert, mit 300 ml Eiswasser gewaschen und getrocknet. Man erhielt 157 g (1.34 mol) L-Norvalin. Das Filtrat (1393 g), das neben 22.5 Gew.-% N-Acetyl-D(L)-norvalin 3.1 Gew.-% L-Norvalin enthielt, wurde auf 60 °C erwärmt und mit 64 g (0.62 mol) Acetanhydrid versetzt. Nach 30 min war in der Lösung kein Norvalin nachweisbar. Es wurden 213 g (1.34 mol) N-Acetyl-D,L-norvalin zugegeben und im Vakuum auf 950 g eingeengt. Diese Lösung wurde mit einem Sambay-Verdampfer bei 170°C und 10 mbar auf 644 g eingeengt. In die noch warme Schmelze wurden 13 g Acetanhydrid eingerührt, 10 min auf 140 °C erhitzt und dann in Wasser gelöst und auf 1.5 l aufgefüllt. Eine chromatographische ee-Bestimmung erbrachte ein Verhältnis D/L von 51.3 % zu 48.7 %. Der Gehalt an N-Acetyldipeptid betrug 1.2 % bez. auf N-Acetyl-norvalin. Nachdem der pH-Wert mit Natronlauge auf 7 gestellt wurde, wurde diese Lösung wie oben beschrieben erneut zur Acylasespaltung eingesetzt. Man erhielt 113 g (0.97 mol) L-Norvalin.

## Patentansprüche

**1.** Verfahren zur Gewinnung von optisch aktiven L-α-Aminocarbonsäuren aus den entsprechenden racemischen D, L-α-Aminocarbonsäuren, bei dem

(a) die D,L-α-Aminocarbonsäuren acetyliert werden;
(b) die im Gemisch der dabei erhaltenen N-Acetyl-D,L-α-aminocarbonsäuren vorliegende N-Acetyl-L-α-aminocarbonsäure enzymatisch in die L-α-Aminocarbonsäure gespalten wird;
(c) die L-α-Aminocarbonsäure aus dem Gemisch abgetrennt wird unter Rückbehalt einer N-Acetyl-D(L)-α-aminocarbonsäuren und eine der gebildeten L-α-Aminocarbonsäure äquivalenten Menge Acetat aufweisenden Lösung; und
(d) die in der Lösung enthaltene N-Acetyl-D(L)-α-aminocarbonsäure racemisiert und zur enzymatischen Spaltung zurückgeführt wird,

**dadurch gekennzeichnet,**
daß die rückbehaltene Lösung aus Schritt c) so eingestellt wird, daß einer aus N-Acetyl-D(L)-α-aminocarbonsäuresalz und freier Essigsäure im Gleichgewicht mit Acetat und freier N-Acetyl-D(L)-α-aminocarbonsäure bestehenden Lösung eine der abgetrennten L-α-Aminocarbonsäure äquimolare Menge N-Acetyl-D,L-α-aminocarbonsäure zugesetzt wird und daß aus dieser Lösung bei einem pH-Wert von zwischen 3 und 8 Essigsäure destillativ abgetrennt wird.

**2.** Verfahren nach Anspruch 1
**dadurch gekennzeichnet,**
daß die Lösung bei einem pH-Wert von zwischen 4,5 und 5,5 bis zur Schmelze eingeengt wird.

**3.** Verfahren nach Anspruch 1 oder 2,
**dadurch gekennzeichnet,**
daß die nach der Einengung erhaltenen Produkte racemisiert und zur enzymatischen Racematspaltung rückgeführt werden.

**4.** Verfahren nach Anspruch 3,
**dadurch gekennzeichnet,**
daß die nicht gespaltene N-Acetyl-D-α-aminocarbonsäure als Salz oder in Mischung zusammen mit der freien Säure in Gegenwart von 1 bis 10 Gew.-% Acetanhydrid bei 100 bis 220 °C racemisiert wird.

**5.** Verfahren nach Anspruch 4,
**dadurch gekennzeichnet,**
daß bei Temperaturen zwischen 130 und 180 °C racemisiert wird.

**6.** Verfahren nach einem oder mehreren der vorherigen Ansprüche,
**dadurch gekennzeichnet,**
daß die N-Acetyl-D,L-α-aminocarbonsäuren zur enzymatischen Spaltung in wäßrigem Medium in Salze überführt werden, und
daß der Lösung vor Durchführung des Schrittes (d) zur Nachacetylierung in ihr verbliebener Reste an L-α-Aminocarbonsäuren ohne Zugabe weiterer Base mindestens 1,1 Mol-Äquivalente, bezogen auf die verbliebene Menge L-α-Aminocarbonsäuren, Acetanhydrid zugegeben wird.

7. Verfahren nach Anspruch 6,
**dadurch gekennzeichnet,**
daß die Lösung zur Acetylierung der nicht abgetrennten L-α-Aminocarbonsäure bei einem pH-Wert zwischen 4 und 8 mit bezogen auf die nicht abgetrennte L-α-Aminocarbonsäure bis zu 3,0 Äquivalenten Acetanhydrid versetzt wird, so daß die resultierende Lösung zur enzymatischen Spaltung eingesetzt werden kann.

8. Verfahren nach einem der vorherigen Ansprüche,
**dadurch gekennzeichnet,**
daß L-Methionin, -Valin, -Phenylalanin und/oder -Norvalin gewonnen werden.

**Claims**

1. Process for obtaining optically active L-α-aminocarboxylic acids from the corresponding racemic D,L-á-aminocarboxylic acids, in which

(a) the D,L-α-aminocarboxylic acids are acetylated;
(b) the N-acetyl-L-α-aminocarboxylic acid present in the mixture of N-acetyl-D,L-α-aminocarboxylic acids thus obtained is broken down enzymatically into the L-α-aminocarboxylic acid;
(c) the L-α-aminocarboxylic acid is separated from the mixture, a solution with N-acetyl-D(L)-α-aminocarboxylic acids and a quantity of acetate equivalent to the L-α-aminocarboxylic acid formed being retained; and
(d) the N-acetyl-D(L)-α-aminocarboxylic acid contained in the solution is racemised and recycled for enzymatic breakdown,

characterised in that
the retained solution from step c) is adjusted in such a way that a quantity of N-acetyl-D,L-α-aminocarboxylic acid equimolar to the separated L-á-aminocarboxylic acid is added to a solution consisting of N-acetyl-D(L)-α-aminocarboxylic acid salt and free acetic acid in equilibrium with acetate and free N-acetyl-D(L)-α-aminocarboxylic acid and that acetic acid is separated from this solution by distillation at a pH of between 3 and 8.

2. Process according to claim 1,
characterised in that
the solution is concentrated to the melt at a pH of between 4.5 and 5.5.

3. Process according to claim 1 or 2,
characterised in that
the products obtained after concentration are racemised and recycled for the enzymatic resolution of racemates.

4. Process according to claim 3,
characterised in that
the N-acetyl-D-α-aminocarboxylic acid that has not been broken down is racemised as the salt or in a mixture together with the free acid in the presence of 1 to 10 wt.% acetic anhydride at 100 to 220°C.

5. Process according to claim 4,
characterised in that
racemisation is performed at temperatures of between 130 and 180°C.

6. Process according to one or more of the above claims,
characterised in that
the N-acetyl-D,L-α-aminocarboxylic acids are converted to salts for enzymatic breakdown in an aqueous medium, and
that at least 1.1 mole equivalents, based on the remaining quantity of L-α-aminocarboxylic acids, of acetic anhydride is [sic] added to the solution before carrying out step (d) for the post-acetylation of residues of L-α-aminocarboxylic acids remaining therein, without the addition of another base.

7. Process according to claim 6,
characterised in that
up to 3.0 equivalents of acetic anhydride, based on the non-separated L-α-aminocarboxylic acid, are added to the

solution for the acetylation of the non-separated L-α-aminocarboxylic acid at a pH of between 4 and 8, so that the resulting solution can be used for enzymatic breakdown.

8. Process according to one of the above claims, characterised in that L-methionine, -valine, -phenylalanine and/or -norvaline are obtained.

**Revendications**

1. Procédé pour l'obtention d'acides L-α-aminocarboxyliques optiquement actifs à partir des acides D,L-α-aminocarboxyliques racémiques correspondants, dans lequel

   (a) on acétyle les acides D,L-α-aminocarboxyliques;
   (b) on dissocie par voie enzymatique l'acide N-acétyl-L-α-aminocarboxylique se trouvant dans le mélange des acides N-acétyl-D,L-α-aminocarboxyliques obtenus en acide L-α-aminocarboxylique
   (c) on sépare l'acide L-α-aminocarboxylique du mélange en gardant une solution contenant des acides N-acétyl-D(L)-α-aminocarboxyliques et une quantité d'acétate équivalente à la quantité d'acide L-α-aminocarboxylique formée ; et
   (d) on racémise l'acide N-acétyl-D(L)-α-aminocarboxylique contenu dans la solution qui est recyclé dans la dissociation enzymatique,

   caractérisé en ce qu'on règle la solution résiduelle provenant de l'étape c) de telle manière qu'on ajoute, à une solution constituée de sel de l'acide N-acétyl-D(L)-α-aminocarboxylique et d'acide acétique libre en équilibre avec de l'acétate et de l'acide N-acétyl-D(L)-α-aminocarboxylique libre, une quantité d'acide N-acétyl-D,L-α-aminocarboxylique équimolaire à la quantité d'acide L-α-aminocarboxylique séparée et en ce qu'on élimine par distillation de l'acide acétique de cette solution, à un pH entre 3 et 8.

2. Procédé selon la revendication 1, caractérisé en ce que la solution est concentrée à un pH entre 4,5 et 5,5 jusqu'au bain de fusion.

3. Procédé selon la revendication 1 ou 2, caractérisé en ce que les produits obtenus après la concentration sont racémisés et recyclés dans la dissociation enzymatique des racémates.

4. Procédé selon la revendication 3, caractérisé en ce que l'acide N-acétyl-D-α-aminocarboxylique qui n'est pas dissocié est racémisé sous forme de sel ou en mélange avec l'acide libre, en présence de 1 à 10 % en poids d'anhydride de l'acide acétique, à 100 jusqu'à 220 °C.

5. Procédé selon la revendication 4, caractérisé en ce qu'on racémise à des températures entre 130 et 180 °C.

6. Procédé selon l'une ou plusieurs des revendications précédentes, caractérisé en ce que les acides N-acétyl-D,L-α-aminocarboxyliques sont transformés en sels pour la dissociation enzymatique en milieu aqueux et en ce qu'on ajoute à la solution, avant la réalisation de l'étape (d) pour la post-acétylation des restes d'acides L-α-aminocarboxyliques encore contenus, sans addition d'autre base, au moins 1,1 moles-équivalents d'anhydride de l'acide acétique, par rapport à la quantité résiduelle d'acides L-α-aminocarboxyliques.

7. Procédé selon la revendication 6, caractérisé en ce que la solution pour l'acétylation de l'acide L-α-aminocarboxylique non séparé est additionnée, à un pH entre 4 et 8, de jusqu'à 3,0 équivalents d'anhydride de l'acide acétique par rapport à l'acide L-α-aminocarboxylique non séparé, de telle manière que la solution obtenue peut être utilisée pour la dissociation enzymatique.

8. Procédé selon l'une ou plusieurs des revendications précédentes, caractérisé en ce qu'on obtient de la L-méthionine, de la L-valine, de la L-phénylalanine et/ou de la L-norvaline.